# EUROPEAN PATENT SPECIFICATION

(11) **EP 1 809 242 B1**
(45) Date of publication and mention of the grant of the patent: **20.03.2013**
(21) Application number: 05826067.0
(22) Date of filing: 09.11.2005
(51) Int. Cl.: A61L 12/14, C11D 3/22, C11D 3/30

(54) **CONTACT LENS DISINFECTION AND PACKAGING SOLUTION**
LÖSUNG ZUR DESINFEKTION UND AUFBEWAHRUNG VON KONTAKTLINSEN
SOLUTION POUR LA DÉSINFECTION ET LA CONSERVATION DE LENTILLES DE CONTACT

(30) Priority: 09.11.2004 US 626371 P
(43) Date of publication of application: 25.07.2007
(73) Proprietor: Abbott Medical Optics Inc., Santa Ana, CA 92705-4933 (US)
(72) Inventor: POWELL, Charles, H., Irvine, CA 92620 (US); HUTH, Stanley, W., Newport Beach, CA 92660 (US)
(74) Representative: HOFFMANN EITLE
(86) International application number: PCT/US2005/040827
(87) International publication number: WO 2006/053164

(56) References cited:
- EP-A- 0 976 407
- WO-A-01/57172
- WO-A-03/072141
- US-A1- 2004 057 980
- US-A1- 2005 196 370

## Description

### Background of the Invention

### Field of the Invention

The invention relates to an improved contact lens disinfection and lens packaging composition wherein the anionic polymeric, eye demulcent Hyaluronic Acid is combined with a cationic monomeric antimicrobial agent, wherein said compositions provide additional comfort and biocompatibility with lenses without significantly affecting the antimicrobial efficacy.

### Description of the Related Art

WO 01/57172 discloses a multi-purpose solution for treating contact lenses, said solution comprising sodium hyaluronate, polyaminopropyl diguanide (polymeric preservative) and a buffer.

Anionic polymers such as hyaluronic acid and carboxymethylcellulose have been noted for their moisturizing and lubricating properties in ophthalmic solutions. Such properties generally result in a reduction in irritation to the eye. Cationic antimicrobial agents have also been noted as beneficial to include in ophthalmic solutions. However, these two components are not viewed as compatible within one solution.

Cationic antimicrobial disinfecting agents have been shown to be compromised in their efficacy in the presence of certain anionic entities. For example, U.S. Patent No. 5,858,346 teaches that poly[hexamethylene biguanide hydrochloride] (PHMB) and other non-oxidative disinfectants (typically cationic entities) are neutralized in their ability to damage cell walls, including cell walls of microorganisms, when combined with Carboxymethylcellulose (CMC) and/or various other negatively charged entities. Although the neutralization of the PHMB or other non-oxidative disinfectants alleviates irritation of the eye, the neutralization also results in loss of antimicrobial efficacy.

Similarly, U.S. Patent No. 5,559,104, to Romeo et al., teaches the use of Cetylpyridinium Chloride (CPC) as an ion-pairing agent for the precipitation and purification of Hyaluronic Acid (HA) in a proposed manufacturing process for the HA biopolymer. Thus, that CPC would retain its antimicrobial activity in the presence of a large excess of Hyaluronic Acid or Carboxymethylcellulose is counterintuitive.

It would be advantageous to have compositions by which a decrease in irritability to the eye is not accompanied by a debilitating loss of antimicrobial efficacy. It is an object of the present invention to provide such compositions as well as methods of their use.

### Detailed Description

A novel contact lens disinfection and lens packaging composition comprising a negatively charged polymeric substance and a cationic monomeric antimicrobial agent is described, as well as its methods of use and preparation. Solutions according to the present invention may be used for effective multipurpose contact lens disinfection compositions and lens packaging solution compositions. The composition provides additional comfort to the eye, and biocompatibility with lenses, without profoundly affecting the efficacy of the antimicrobial agent.

It has been discovered that certain naturally occurring and/or synthetic anionic polymeric substances previously presumed to be incompatible with cationic monomeric or dimeric antimicrobial agents do not profoundly inhibit the antimicrobial activity of said cationic antimicrobial agents.

The compositions are compatible with typical and new additives to contact lens disinfection compositions, such as typical buffer systems, surfactants, demulcents, amino acids, and viscosity imparting agents, including hydroxypropylmethyl cellulose (HPMC).

The composition is comprised of specific concentration ranges of said cationic monomeric antimicrobial agents and said water-soluble anionic polymeric substance.

The anionic polymeric substance has a sufficiently low charge density such that the interaction between said anionic substance and said cationic monomeric antimicrobial agent is sufficiently weak, and does not profoundly inhibit the microbiological activity of said antimicrobial agent.

The composition according to the invention comprises 0.001% to 1% w/v hyaluronic acid, from 0.5 to 5 ppm cetylpyridinium chloride and a buffer.

The aqueous medium of the present compositions includes a buffer component which is present in an amount effective to maintain the pH of the product in the desired range. The present compositions preferably include an effective amount of a tonicity adjusting component to provide the compositions with the desired tonicity.

The aqueous phase or component in the present compositions may have a pH which is compatible with the intended use, and is often in the range of about 4 to about 10. A variety of conventional buffers may be employed, such as phosphate, borate, citrate, acetate, histidine, tris, bis-tris and the like and mixtures thereof. Borate buffers include boric acid and its salts, such as sodium or potassium borate. Potassium tetraborate or potassium metaborate, which produce boric acid or a salt of boric acid in solution, may also be employed. Boric acid may be combined with a geminal cis diol-containing substance, such as sorbitol, to effectively lower the pK of boric acid, thereby enhancing the buffer capacity of borate at the desirable physiologically optimal pH range. Hydrated salts such as sodium borate decahydrate can also be used. Phosphate buffers include phosphoric acid and its salts; for example, M₂HPO₄ and MH₂PO₄, wherein M is an alkali metal such as sodium and potassium. Hydrated salts can also be used. In one embodiment of the present invention, Na₂HPO₄ 7H₂O and NaH₂PO₄ H₂O are used as buffers. The term phosphate also includes compounds that produce phosphoric acid or a salt of phosphoric acid in solution. Additionally, organic counter-ions for the above buffers may also be employed. The concentration of buffer generally varies from about 0.01 to 2.5 w/v% and more preferably varies from about 0.05 to about 0.5 w/v %.

The type and amount of buffer are selected so that the formulation meets the functional performance criteria of the composition, such as physicochemical attributes and shelf life stability, antimicrobial efficacy, buffer capacity and the like factors. The buffer is also selected to provide a pH, which is compatible with the eye and any contact lenses with which the composition is intended for use. Generally, a pH close to that of human tears, such as a pH of about 7.45, is very useful, although a wider pH range from about 6 to about 9, more preferably about 6.5 to about 8.5 and still more preferably about 7.0 to about 8.0 is also acceptable. In one embodiment, the present composition has a pH of about 7.4.

The osmolarity of the present compositions may be adjusted with tonicity agents to a value which is compatible with the intended use of the compositions. For example, the osmolarity of the composition may be adjusted to approximate the osmotic pressure of normal tear fluid, which is equivalent to about 0.9 w/v% of sodium chloride in water. Examples of suitable tonicity adjusting agents include, without limitation: chloride salts of sodium, potassium, calcium and magnesium; dextrose; glycerin; propylene glycol; mannitol; sorbitol and the like; and mixtures thereof. In one embodiment, a combination of sodium chloride and potassium chloride are used to adjust the tonicity of the composition.

Tonicity agents are typically used in amounts ranging from about 0.001 to 2.5 w/v%. These amounts have been found to be useful in providing sufficient tonicity for maintaining ocular tissue integrity. Preferably, the tonicity agent(s) will be employed in an amount to provide a final osmotic value of 150 to 450 mOsm/kg, more preferably between about 220 to about 350 mOsm/kg and most preferably between about 270 to about 310 mOsm/kg.

In another embodiment, the composition preferably comprises 0.001% to 1% w/v) hyaluronic acid, from 0.5 to 5 ppm cetylpyridinium chloride and an aqueous medium comprising an alkali metal salt, such as sodium chloride, and a buffer agent, such as phosphoric acid and its related salts, boric acid and its related salts, or tri(2-hydroxymethyl)methylamine (Tris) and its related salts, such as to render the final osmolarity between 220 and 350 mOsm/kg and a preferred final pH between 6.5 and 8.5.

In another embodiment, the composition preferably comprises 0.001 % to 1% w/v hyaluronic acid, from 0.5 to 5 ppm cetylpyridinium chloride and an aqueous medium comprising one or more alkali metal salts, such as sodium chloride, and a buffer agent, such as phosphoric acid and its related salts, boric acid and its related salts, or tri(2-hydroxymethyl)methylamine (Tris) and its related salts, such as to render the final osmolarity between 280 and 310 mOsm/kg and a preferred final pH between 7.0 and 8.0.

In another embodiment, the composition preferably comprises 0.001% to 1% w/v hyaluronic acid, from 0.5 to 5 ppm cetylpyridinium chloride, a buffer and an aqueous medium comprising one or more surfactants, such as polyethylene oxide (PEO), polypropylene oxide (PPO), or block copolymers comprised of combinations of these, to achieve enhanced cleaning during lens treatment without significant loss of antimicrobial activity of the disinfecting agent(s). In an alternative embodiment, other PEO-PPO copolymer formats, wherein PEO-PPO chains are anchored to the functional groups of a poly-functional entity, such as the acetyl groups of ethylenediaminetetraacetic acid, as a means of increasing the molecular size of the surfactant, are used as the surfactant entity for said composition.

In another embodiment, the composition preferably comprises 0.001% to 1% w/v hyaluronic acid, from 0.5 to 5 ppm cetylpyridinium chloride, a buffer and an aqueous medium comprising one or more demulcents, such as propylene glycol, poly(hydroxypropylmethyl cellulose) or a short-chain polyethylene oxide, added preferably between from about as 0.05% to 1% w/v, to achieve enhanced cleaning and water retention of lenses, without significant loss of antimicrobial performance of the disinfecting agent(s).

In another embodiment, the composition preferably comprises 0.001% to 1% w/v hyaluronic acid, from 0.5 to 5 ppm cetylpyridinium chloride, a buffer and an aqueous medium comprising one or more chelating agents, such as ethylenediaminetetraacetic acid or one of its salts is added at an appropriate concentration, preferably from about 0.001% to about 0.01% w/v to insure solubilization of calcium from tear components during lens treatment with said contact lens disinfection composition.

In another embodiment, the composition preferably comprises 0.001% to 1% w/v hyaluronic acid, from 0.5 to 5 ppm cetylpyridinium chloride, a buffer and an aqueous medium comprising one or more amino acids, such as taurine, serine and glycine, added at a concentration to provide potential health benefit, preferably from about 0.001% to about 0.1% w/v, while not significantly altering the antimicrobial performance of the disinfecting agent(s).

In another embodiment, the composition preferably comprises 0.001% to 1% w/v) hyaluronic acid, from 0.5 to 5 ppm cetylpyridinium chloride, a buffer and an aqueous medium comprising one or more vitamins or vitamin-related substances, such as tocopheryl polyethylene glycol succinate (TPGS) added at an appropriate level, preferably from about 0.0001% to about 0.1% w/v, to impart additional surfactant capacity to said disinfection composition.

In another embodiment, the composition preferably comprises 0.001% to 1% w/v hyaluronic acid, from 0.5 to 5 ppm cetylpyridinium chloride, a buffer and an aqueous medium comprising one or more viscosity modifying agents or components, such as cellulose polymers, including hydroxypropylmethyl cellulose (HPMC), hydroxyethyl cellulose (HEC), ethyl hydroxyethyl cellulose, hydroxypropyl cellulose, methyl cellulose and carboxymethyl cellulose; carbomers (e.g. carbopol. RTM); polyvinyl alcohol; polyvinyl pyrrolidone; alginates; carrageenans; and guar, karaya, agarose, locust bean, tragacanth and xanthan gums. Such viscosity modifying components are employed, if at all, in an amount effective to provide a desired viscosity to the present compositions. The concentration of such viscosity modifiers will typically vary between about 0.01 to about 5% w/v of the total composition, although other concentrations of certain viscosity modifying components may be employed.

In another embodiment, the composition preferably comprises 0.001% to 1% w/v hyaluronic acid, from 0.5 to 5 ppm cetylpyridinium chloride, a buffer and an aqueous medium, wherein the hyaluronic acid has a molecular weight preferably between 70,000 and 4 million daltons and more preferably a molecular weight between 700,000 and 2 million daltons.

In another embodiment, hyaluronic acid is preferably added at a concentration between about 0.005% to about 0.2% w/v.

In another embodiment, hyaluronic acid has a molecular weight preferably between 750,000 and 2 million daltons and is preferably added at a concentration between about 0.005% to about 0.2% w/v.

The concentration of hyaluronic acid depending on its molecular weight, is 0.001% to 1% w/v and, preferably, 0.01% to 0.1%.

In another embodiment. cetylpyridinium chloride is preferably added at a concentration of about 1 to about 3 ppm, and more preferably at a concentration of about 1.3 to about 2.3 ppm.

In another embodiment, cetylpyridinium chloride is preferably added at a concentration of about 1 to about 3.5 ppm.

Methods for treating a contact lens using the herein described compositions are included within the scope of the invention. In general, such methods comprise contacting a contact lens with such a composition at conditions effective to provide the desired treatment to the contact lens.

The contact lens can be contacted with the composition, often in the form of a liquid aqueous medium, by immersing the lens in the composition. During at least a portion of the contacting time period, the composition containing the contact lens can be agitated, for example, by shaking the container containing the composition and contact lens, to facilitate the contact lens treatment, for example, the removal of deposit material from the lens. Before or after such contacting step, in contact lens cleaning, the contact lens may be manually rubbed to remove further deposit material from the lens. The cleaning method can also include rinsing the lens prior to or after the contacting step and/or rinsing the lens substantially free of the composition prior to returning the lens to the wearer's eye.

It should be clearly understood that the following examples are illustrative only and are not intended to limit the scope of the present invention.

### EXAMPLES

### Example 1

An example of the composition according to the present invention was prepared as shown in Table 1. This composition, which surprisingly meets the disinfecting standards set forth for a 'no rub' solution, may provide additional comfort to the eye, and biocompatibility with lenses. It is noted that the anionic polymeric substance (in this example, HA), does not profoundly affect the efficacy of the antimicrobial agent.

As shown in Table 1 below, Cetylpyridinium Chloride (CPC) at various concentrations is found to be somewhat moderated in antimicrobial performance in the presence of 0.075% Hyaluronic Acid (HA) with average molecular weight (MW) of about 1.7 million (M) daltons, as compared to 0.2% HPMC, when using a Tris buffered contact lens disinfection composition. However, the slight lowering of activity is deemed minor with respect to the ability to achieve sufficient kill at low concentration for a robust disinfection system for contact lenses. Table 1 shows that CPC activity toward three bacteria and two fungi (*C. Albicans* and *F*. *Salami*) is lowered on average only by about one-half log in the sum microbial kill level of five common microbes, relative to the HPMC-containing formula. The loss of some activity toward *S*. *aureus* indicates only slight ion pairing between CPC and this relatively high molecular weight HA material. The fact that only slight ion pairing is seen is contrary to the teachings of the prior art.

**Table 1: Antimicrobial activity toward five organisms versus CPC concentration in a Tris buffered formulation containing either 0.2% HPMC or 0.075% HA (MW = 1.7M daltons)**

| **Ingredient** | **HA Formulations** | | | | | **HPMC Formulations** Reference Examples | | | | | **Complete®** |
|---|---|---|---|---|---|---|---|---|---|---|---|
| **CPC (ppm)** | 1.00 | 1.25 | 1.50 | 1.75 | 2.00 | 1.00 | 1.25 | 1.50 | 1.75 | 2.00 | Lot 30616 (Control) |
| **HA (1.7M) (%)** | 0.075 | 0.075 | 0.075 | 0.075 | 0.075 | | | | | | |
| **HPMC (%)** | | | | | | 0.2 | 0.2 | 0.2 | 0.2 | 0.2 | |
| **Pluronic® F-87 (%)** | 0.05 | 0.05 | 0.05 | 0.05 | 0.05 | 0.05 | 0.05 | 0.05 | 0.05 | 0.05 | |
| **NaCl (%)** | 0.59 | 0.59 | 0.59 | 0.59 | 0.59 | 0.59 | 0.59 | 0.59 | 0.59 | 0.59 | |
| **KCl (%)** | 0.14 | 0.14 | 0.14 | 0.14 | 0.14 | 0.14 | 0.14 | 0.14 | 0.14 | 0.14 | |
| **Propylene glycol (%)** | 0.5 | 0.5 | 0.5 | 0.5 | 0.5 | 0.5 | 0.5 | 0.5 | 0.5 | 0.5 | |
| **EDTA (%)** | 0.01 | 0.01 | 0.01 | 0.01 | 0.01 | 0.01 | 0.01 | 0.01 | 0.01 | 0.01 | |
| **Taurine (%)** | 0.05 | 0.05 | 0.05 | 0.05 | 0.05 | 0.05 | 0.05 | 0.05 | 0.05 | 0.05 | |
| **Tris HCl (%)** | | 0.055 | 0.055 | 0.055 | 0.055 | 0.055 | 0.055 | 0.055 | 0.055 | 0.055 | |
| **Tris (%)** | 0.021 | 0.021 | 0.021 | 0.021 | 0.021 | 0.021 | 0.021 | 0.021 | 0.021 | 0.021 | |

| **Organism** | **Log Drop in Live Organisms** | | | | | | | | | | |
|---|---|---|---|---|---|---|---|---|---|---|---|
| **S.marcescens 13880** | 1.59 | 2.17 | 2.82 | 3.29 | 3.53 | 1.03 | 2.69 | 3.09 | 3.87 | 3.74 | 4.65 |
| ***S*. *aureus* 6538** | 2.38 | 2.26 | 3.21 | 3.41 | 3.76 | 3.61 | 2.86 | 3.98 | 4.15 | 4.76 | 4.76 |
| ***P. aeruginosa* 9027** | 4.58 | 4.58 | 4.58 | 4.58 | 4.58 | 4.28 | 4.58 | 4.58 | 4.58 | 4.58 | 4.58 |
| ***C. albicans* 10231** | 1.20 | 1.87 | 2.36 | 2.56 | 3.15 | 1.43 | 2.16 | 2.31 | 3.14 | 2.23 | 1.57 |
| ***F. solani* 36031** | 0.68 | 0.55 | 0.80 | 0.70 | 0.77 | 0.70 | 0.42 | 0.41 | 0.51 | 0.51 | 2.37 |
| **Sum** | **10.43** | **11.43** | **13.77** | **14.54** | **15.79** | **11.05** | **12.71** | **14.37** | **16.25** | **15.82** | **17.93** |

### Example 2

Further examples of compositions according to the present invention were prepared as shown in Tables 2A and 2B. Again, the cationic antimicrobial does not profoundly affect the efficacy of the antimicrobial agent.

Comparing Table 1 and Table 2B, Alexidine Dihydrochloride (ALX) is demonstrated to exhibit more antimicrobial efficacy (AME) activity on a weight-per-volume basis than CPC, when Tris buffer with 0.2% HPMC is used as the base formula. However, comparison of Table 1 and Table 2A indicates that, in the presence of HA, CPC retains better activity toward *S*. *Aureus* than ALX. Further comparison of Table 1 and Table 2A shows that borate buffered CPC containing 0.075% HA (MW=1.7M) exhibits much higher activity than Tris buffered CPC containing the same 0.075% HA. Although both Tris buffered and borate buffered "Placebos" (formulas containing all ingredients except the antimicrobial agent) show little or no AME activity, borate provides a demonstrable synergism with CPC that substantially boosts the overall AME activity. Comparing Table 2A and Table 2B shows that the AME activity of ALX in Tris buffer is also moderated when 0.05% HA (MW = 1.7M) is used as the demulcent instead of 0.2% HPMC. Thus, both CPC and ALX exhibit slight reduction in activity when HA having a MW of 1.7M is employed as the demulcent. The loss of only some activity again indicates only slight ion pairing between CPC and this relatively high molecular weight HA material. The fact that only slight ion pairing is seen is contrary to the teachings of the prior art.

**Table 2A: Antimicrobial activity toward five organisms in HA-containing Tris and Borate buffered systems, respectively, versus ALX or CPC concentration**

| **Ingredient** | **ALX Formulations** Reference Examples | | | | | **CPC Formulations** | | | | | | Complete® |
|---|---|---|---|---|---|---|---|---|---|---|---|---|
| ALX (ppm) | 1.25 | 1.50 | 1.75 | 2.00 | 2.50 | | | | | | | |
| CPC (ppm) | 0.05 | | | | | 1.00 | 1.50 | 1.70 | 1.85 | 2.00 | 2.15 | Exp:Sep.05 Lot 27321 (Control) |
| HA (M.W.=1.7M) (%) | | 0.05 | 0.05 | 0.05 | 0.05 | 0.075 | 0.075 | | | 0.075 | 0.075 | |
| Tris HCl (%) | 0.055 | 0.055 | 0.055 | 0.055 | 0.055 | | | 0.075 | | | | |
| Tris Base (%) | 0.021 | 0.021 | 0.021 | 0.021 | 0.021 | | | | | | | |
| NaCl (%) | 0.59 | 0.59 | 0.59 | 0.59 | 0.59 | 0.59 | 0.59 | 0.59 | 0.59 | 0.59 | 0.59 | |
| KCl (%) | 0.14 | 0.14 | 0.14 | 0.14 | 0.14 | 0.14 | 0.14 | 0.14 | 0.14 | 0.14 | 0.14 | |
| EDTA (%) | 0.01 | 0.01 | 0.01 | 0.01 | 0.01 | 0.01 | 0.01 | 0.01 | 0.01 | 0.01 | 0.01 | |
| Taurine (%) | 0.05 | 0.05 | 0.05 | 0.05 | 0.05 | 0.05 | 0.05 | 0.05 | 0.05 | 0.05 | 0.05 | |
| Pluronic® F87 (%) | | 0.05 | 0.05 | 0.05 | 0.05 | 0.05 | 0.05 | 10.05 | 0.05 | 0.05 | 0.05 | |
| Propylene Glycol (%) | 0.05 | 0.5 | 0.5 | 0.5 | 0.5 | | | | | | | |
| PEG 400 (%) | 0.5 | | | | | 0.1 | 0.1 | 0.1 | 0.1 | 0.1 | 0.1 | |
| Boric acid (%) | | | | | | 0.6 | 0.6 | 0.6 | 0.6 | 0.6 | 0.6 | |
| NaOH , 1 N (%) | | | | | | 0.675 | 0.675 | 0.675 | 0.675 | 0.675 | 0.675 | |

| **Organism** | **Log dop in viable organisms at 6 hours after -1xE5 innoculum of specified organism:** | | | | | | | | | | | |
|---|---|---|---|---|---|---|---|---|---|---|---|---|
| *S. marcescens* 138801 | 3.15 | 3.43 | 4.60 | 4.60 | 4.60 | 3.00 | 3.14 | 3.32 | 3.01 | 4.60 | 3.70 | 4.60 |
| *S. marcescens (#2)* | 3.59 | 4.59 | 4.59 | 4.59 | 4.59 | 1.48 | 4.59 | 4.59 | 4.59 | 3.29 | 3.70 | 3.29 |
| *SM average* | 3.37 | 4.01 | 4.60 | 4.60 | 4.60 | 2.23 | 3.87 | 3.96 | 3.80 | 3.95 | 3.50 | 3.95 |
| *S. aureus* 6538 | 1.53 | 2.59 | 3.07 | 3.16 | 3.21 | 3.01 | 2.40 | 3.54 | 3.00 | 3.94 | 4.15 | 4.85 |
| *S. aureus* (#2) | 1.29 | 1.95 | 2.67 | 3.04 | 3.68 | 2.19 | 4.05 | 3.65 | 3.70 | 4.95 | 4.05 | 4.95 |
| *SA average* | 1.41 | 2.27 | 2.87 | 3.10 | 3.45 | 2.60 | 3.23 | 3.60 | 3.35 | 4.45 | 4.10 | 4.90 |
| *P. aeruginosa* 9027 | 4.59 | 4.59 | 4.59 | 4.59 | 4.59 | 4.59 | 4.59 | 4.59 | 4.59 | 4.59 | 4.59 | 4.59 |
| *C. albicans* 10231 | 0.88 | 0.89 | 1.31 | 1.47 | 1.79 | 1.73 | 2.61 | 2.26 | 2.86 | 3.55 | 2.91 | 1.42 |
| *F. solani* 36031 | 1.93 | 3.14 | 3.71 | 4.19 | 3.58 | 2.19 | 3.11 | 3.07 | 4.19 | 3.58 | 3.41 | 3.07 |
| Sum of Organisms | 12.18 | 14.90 | 17.07 | 17.94 | 18.00 | 13.34 | 17.40 | 17.47 | 18.79 | 20.11 | 18.50 | 17.93 |

**Table 2B: Antimicrobial activity in Tris buffered formulations containing 0.2% HPMC toward five organisms versus Alexidine Dihydrochloride concentration**

| **Formulation** | **HPMC Formulations** Reference Examples | | | **Complete®** |
|---|---|---|---|---|
| ALX (ppm) | 1.50 | 2.00 | 2.50 | Lot 27321 |
| HPMC(%) | 0.20 | 0.20 | 0.20 | |
| Pluronic® F-87 (%) | 0.05 | 0.05 | 0.05 | |
| NaCl (%) | 0.59 | 0.59 | 0.59 | Exp: Sep.05 |
| KCl (%) | 0.14 | 0.14 | 0.14 | |
| Propylene glycol (%) | 0.50 | 0.50 | 0.50 | (Control) |
| EDTA (%) | 0.01 | 0.01 | 0.01 | |
| Taurine (%) | 0.05 | 0.05 | 0.05 | |
| Tris HCl (%) | 0.055 | 0.055 | 0.055 | |
| Tris Base (%) | 0.021 | 0.021 | 0.021 | |

| **Organism** | **Log drop in live organism** @ **6 hrs.** | | | |
|---|---|---|---|---|
| *S. marcescens* 13880 | 4.83 | 4.83 | 3.75 | 3.53 |
| *S. aureus* 6538 | 3.69 | 4.51 | 4.81 | 4.51 |
| *P. aeruginosa* 9027 | 4.73 | 4.73 | 4.73 | 4.73 |
| *C. albicans* 10231 | 1.18 | 1.72 | 1.87 | 1.49 |
| *F. solani* 36031 | 2.18 | 2.73 | 4.18 | 2.97 |
| **Sum** | **16.61** | **18.52** | **19.34** | **17.23** |

### Example 3

Tests were performed to determine whether the molecular weight of the particular anionic polymer had any effect on the efficacy of the solution. Table 3 shows the effect of HA molecular weight upon the antimicrobial activity of three disinfection chemical entities: PHMB, ALX and CPC. Complete@ Moisture Plus™ multi-purpose solution (containing 0.15% HPMC and 1.0 ppm PHMB) serves as the non-HA control for the PHMB-HA formulation, while the CPC non-HA control is presented in Table 3 (at 2.0 ppm CPC and 0.15% HPMC). The ALX non-HA control for 2.0 ppm ALX is found in Table 2B. The data demonstrate that HA having a molecular weight of 1.5M or 0.8M has no measurable reductive effect on the antimicrobial efficacy of these three chemical entities, relative to HPMC at 0.15% or 0.2%. The relatively high molecular weight version of HA, 1.7M, is seen to produce the greatest inhibition of antimicrobial efficacy toward PHMB, where the sum of the kill level is reduced by about 4 log relative to the non-HA containing control and the lower molecular weight HA PHMB compositions. The ALX compositions are also significantly affected in antimicrobial efficacy by the highest presented molecular weight version of HA (1.7M), loosing about 2 log of activity relative to the lower molecular weight entities. The CPC shows the least effect on efficacy by the presence of the largest tested HA (1.7M), losing less than 1 log of sum activity. The loss of only some activity again indicates only slight ion pairing between CPC and this relatively high molecular weight HA material. The fact that only slight ion pairing is seen is contrary to the teachings of the prior art.

**Table 3: Antimicrobial activity of three different disinfection chemical entities in Tris buffered formulations containing HA of three different molecular weights: 0.8M, 1.5M and 1.7M**

| **Ingredient** | **CPC Formulations** | | | | | | | **Alex. Formulations** Reference Examples | | | **PHMB Formulations** Reference Examples | | | **Complete®** |
|---|---|---|---|---|---|---|---|---|---|---|---|---|---|---|
| PHMB (ppm) | | | | | | | | | | | 1.0 | 1.0 | 1.0 | Lot 27321 Exp: 9/05 |
| Alexidine (ppm) | | | | | | | | 2.0 | 2.0 | 2.0 | | | | |
| CNC (ppm) | 1.5 | 1.5 | 1.5 | 2.0 | 2.0 | 2.0 | 2.0 Reference Examples | | | | | | | |
| HA(MW=0.8M) (%) | 0.05 | | | 0.05 | | | | 0.05 | | 0.05 | 0.05 | | | (Control) |
| HA (MW=1.5M) (%) | | | | | 0.05 | | | | 0.05 | | | | | |
| HA (MW = 1.7M) (%) | | | 0.055 | | | 0.05 | | | | | | 0.05 | 0.05 | |
| HPMC (%) | | | | | | | 0.15 | | | | | | | |
| Tris HCl (%) | 0.055 | 0.055 | 0.055 | 0.055 | 0.055 | 0.055 | 0.055 | 0.055 | 0.055 | 0.055 | 0.055 | 0.05 | 0.055 | |
| Tris Base (%) | 0.021 | 0.021 | 0.021 | 0.021 | 0.021 | 0.021 | 0.021 | 0.021 | 0.021 | 0.021 | 0.021 | 0.021 | 0.021 | |
| NaCl (%) | 0.59 | 0.59 | 0.59 | 0.59 | 0.59 | 0.59 | 0.59 | 0.59 | 0.59 | 0.59 | 0.59 | 0.59 | 0.59 | |
| KCl (%) | 0.14 | 0.14 | 0.14 | 0.14 | 0.14 | 0.14 | 0.14 | 0.14 | 0.14 | 0.14 | 0.14 | 0.14 | 0.14 | |
| Edetate Disodium (%) | 0.01 | 0.01 | 0.01 | 0.01 | 0.01 | 0.01 | 0.01 | 0.01 | 0.01 | 0.01 | 0.01 | 0.01 | 0.01 | |
| Taurine (%) | 0.05 | 0.05 | 0.05 | 0.05 | 0.05 | 0.05 | 0.05 | 0.05 | 0.05 | 0.05 | 0.05 | 0.05 | 0.05 | |
| *Pluronic® F87 (%)* | 0.05 | 0.05 | 0.05 | 0.05 | 0.05 | 0.05 | 0.05 | 0.05 | 0.05 | 0.05 | 0.05 | 0.05 | 0.05 | |
| Propylene Grycol | 0.5 | 0.5 | 0.5 | 0.5 | 0.5 | 0.5 | 0.5 | 0.5 | 0.5 | 0.5 | 0.5 | 0.5 | 0.5 | |

| **Organism** | **Log dop in viable organisms at 6 hours alter ∼1xE5 innoculum of specified organism:** | | | | | | | | | | | | | |
|---|---|---|---|---|---|---|---|---|---|---|---|---|---|---|
| *S. marceseens 13880* | 2.74 | 3.25 | 3.87 | 3.67 | 3.7 | 4.10 | 4.03 | 4.88 | 4.88 | 4.88 | 4.88 | 4.88 | 3.64 | 4.88 |
| *S. aureus* 6538 | 4.2 | 4.68 | 3.2 | 4.2 | 4.68 | 3.98 | 3.57 | 4.38 | 3.78 | 3.30 | 4.68 | 4.68 | 3.04 | 4.68 |
| *P. aeruginosa 9027* | 4.56 | | 4.56 | 4.56 | 3.71 | 4.56 | 4.56 | 4.56 | 4.56 | 4.56 | | 4.56 | 4.56 | 4.56 |
| *C. albicans 10231* | 2.87 | 3.24 | 2.94 | 3.10 | 2.85 | 2.8 | 3.39 | 1.6 | 1.88 | 1.74 | 1.35 | 1.52 | 0.46 | 1.83 |
| *F. solani* 36031 | 1.89 | 1.76 | 1.64 | 2.50 | 2.60 | 2.44 | 1.82 | 3.75 | 3.87 | 2.87 | 2.79 | 2.87 | 2.54 | 2.93 |
| **Sum** | **16.26** | **17.48** | **16.01** | **18.03** | **17.54** | **17.88** | **17.371** | **19.17** | **18.95** | **17.35** | **18.26** | **18.51** | **14.24** | **18.88** |

### Example 4 (Reference Example)

The following example shows the effect on antimicrobial activity of a monomeric antimicrobial agent, CPC, of the addition of 0.5% of a commercially obtained (from Hercules Inc., Wilmington, DE) low viscosity form of carboxymethylcellulose. The resulting Test solution (Solution #4 in Table 4) exhibited a viscosity of 3.5 centipoises. The activity against three key organisms of the Test solution was evaluated in a series of increasing concentrations of CPC in solutions containing 0.2% HPMC instead of 0.5% CMC. The Test solution exhibited slightly higher activity against *S*. *marcescens* than the comparison solution having a slightly higher (but comparable) level of CPC (#5). The Test solution also meets the stand-alone criteria at this concentration of CPC. The Test and Control solutions only differ in the demulcent identity (CMC vs. HPMC) and concentration of CPC. The CPC level in the Test solution is bracketed by the CPC levels in Solutions #3 and #5. While the *S*. *aureus* log reduction is lower for the Test solution than for the bracketing concentrations of CPC control solutions, this degree of inhibition of activity against this organism is not prohibitive for the present applications.

**Table 4: Relative antimicrobial activity of a Test solution (#4) containing CPC in combination with low-viscosity CMC in a series of increasing concentrations of CPC in combination with HPMC (Reference Example)**

| **Solution** #/ **Ingredient** | **1** | **2** | **3** | **4** 4 | **5** |
|---|---|---|---|---|---|
| NaCl (%) | 0.55 | 0.55 | 0.55 | 0.55 | 0.55 |
| KCl (%) | 0.14 | 0.14 | 0.14 | 0.14 | 0.14 |
| PF-87 (%) | 0.05 | 0.05 | 0.05 | 0.55 | 0.55 |
| Taurine (%) | 0.05 | 0.05 | 0.05 | 0.05 | 0.05 |
| PEG-400 (%) | 0.10 | 0.10 | 0.10 | 0.10 | 0.10 |
| Sorbitol (%) | 0.20 | 0.20 | 0.20 | 0.20 | 0.20 |
| Boric Acid (%) | 0.10 | 0.10 | 0.10 | 0.10 | 0.10 |
| EDTA (%) | 0.01 | 0.01 | 0.01 | 0.01 | 0.01 |
| HPMC (%) | 0.20 | 0.20 | 0.20 | 0 | 0.20 |
| CMC (Low-Vis) (%) | 0 | 0 | 0 | 0.50 | 0.00 |
| CPC (ppm) | 0.595 | 0.809 | 1.03 | 0.50 1.25 | 1.35 |

| **Organism** | **Log drop** @ **6 hours** | | | | |
|---|---|---|---|---|---|
| *S*. *marcescens* 13880 | 0.81 | 1.68 | 2.34 | 3.40 | 3.24 |
| *S*. *aureus* 6538 | 0.47 | 2.51 | 5.10 | 3.59 | 5.18 |
| *F*. *solani* 36031 | 0.98 | 1.36 | 1.66 | 1.92 | 2.22 |
| **Sum** | 2.26 | 5.55 | 9.10 | 8.91 | 10.64 |

## Claims

1. Multipurpose contact lens disinfection and lens packaging solution composition comprising hyaluronic acid, from 0.5 to 5 ppm cetylpyridinium chloride, and a buffer, wherein the hyaluronic acid is present in an amount ranging from 0.001% to 1% w/v.

2. The multipurpose contact lens disinfection and lens packaging solution composition according to claim 1, wherein the hyaluronic acid has a molecular weight of from 70,000 to 4 million Daltons.

## Patentansprüche

1. Mehrzweckkontaktlinsendesinfektions- und Linsenverpackungslösungszusammensetzung, die Hyaluronsäure, von 0,5 bis 5 ppm Cetylpyridiniumchlorid und einen Puffer umfasst, wobei die Hyaluronsäure in einer Menge im Bereich von 0,001 % bis 1 % W/V vorliegt.

2. Mehrzweckkontaktlinsendesinfektions- und Linsenverpackungslösungszusammensetzung gemäß Anspruch 1, wobei die Hyaluronsäure ein Molekulargewicht von 70.000 bis 4 Millionen Dalton hat.

## Revendications

1. Composition de solution de désinfection et de conservation de lentille de contact à usages multiples comprenant un acide hyaluronique, de 0,5 à 5 ppm de chlorure de cétylpyridinium, et un tampon, où l'acide hyaluronique est présent en une quantité allant de 0,001% à 1% w/v.

2. Composition de solution de désinfection et de conservation de lentilles de contact à usages multiples selon la revendication 1, dans lequel l'acide hyaluronique présente un poids moléculaire allant de 70000 à 4 millions de Daltons.
